# EUROPEAN PATENT APPLICATION

(11) **EP 2 465 346 A2**
(43) Date of publication of application: **20.06.2012**
(21) Application number: 10808013.6
(22) Date of filing: 12.08.2010
(51) Int. Cl.: A01K 67/02, G01N 33/00

(54) **HUMANISED PSORIASIS MODEL**

(30) Priority: 14.08.2009 ES 200930599
(71) Applicant: CENTRO DE INVESTIGACIONES ENERGETICAS MEDIOAMBIENTALES Y TECNOLOGICAS (C.I.E.M.A.T.), 28040 Madrid (ES); Centro Comunitario De Sangre Y Tejidos De Asturias, 33006 Oviedo (ES)
(72) Inventor: GUERRERO ASPIZUA, Sara, E-28040 Madrid (ES); CARRETERO TRILLO, Marta, E-28040 Madrid (ES); GARCÍA DIEZ, Marta, E-28040 Madrid (ES); JORCANO NOVAL, José Luis, E-28040 Madrid (ES); LARCHER LAGUZZI, Fernando, E-28040 Madrid (ES); DEL RIO NECHAEVSKY, Marcela, E-28040 Madrid (ES); MEANA INFIESTA, Álvaro, E-33006 Oviedo (asturias) (ES)
(74) Representative: Pons Ariño, Angel
(86) International application number: PCT/ES2010/070551
(87) International publication number: WO 2011/018545

(57) **Abstract**

The invention relates to a novel humanised animal psoriasis model. Said model is generated by means of the grafting of humanised skin equivalents in the animal, the injection of T lymphocytes and cytokines involved in the generation of the disease in humans, and the implementation of the tape-stripping technique for damaging said grafted equivalents. Said model can be used to study the disease and to identify and evaluate the efficiency of the compounds in controlling said disease.

## Description

The present invention pertains to the field of biotechnology and relates to a method for inducing a psoriatic phenotype in a non-human mammal, to the animal model of psoriasis obtainable by said method and the use thereof for the identification and evaluation of the efficacy of new treatments.

### PRIOR STATE OF THE ART

Psoriasis is a disease that is widespread throughout the world, and affects about 2 % of the population, although its distribution is not homogeneous. The disease is characterised by the appearance of red-colour papular-squamous plaques covered with scales. They are mostly found in body areas subjected to a high friction, such as, for example, the outer part of the knees and elbows, or the lumbosacral area. Moreover, the Koebner phenomenon tends to occur, which is characterised by the appearance of plaques in areas subjected to pressure or which undergo a trauma.

As regards its etiology, there are increasingly signs that point to a multi-factor origin of the disease. The latter seems to be determined by both genetic and environmental elements, as well as immunological and epidermal factors. Within the immunological component, it has been demonstrated that the elements with the greatest significance in the induction and progression of the inflammation underlying the disease are T lymphocytes. There is a close relationship between the Th1/Th2 disequilibrium and the onset of certain autoimmune diseases. It is well-documented that activated T cells are partly responsible for the phenotypic changes observed in psoriatic skin. Amongst them, type 1 cells seem to play an essential role in the pathogenesis of psoriasis (Schlaak et al. 1994. J Invest Dermatol; 102: 145-149). Another sub-population of T cells, called Th17, has been recently characterised as a different sub-population of the sub-populations of Th1 and Th2 cells. Initially, it was reported that these cells played significant roles in the immunopathology of different experimental autoimmune mouse models (experimental autoimmune encephalomyelitis (EAE); collagen-induced arthritis (CIA)) (Cua et al. 2003 Nature; 421: 744-748; Murphy et al. 2003. J Exp Med; 198: 1951-1957) and, more recently, they have been identified in several human pathological conditions (contact dermatitis (RA), Crohn's disease (CD) and psoriasis) (Albanesi et al. 1999. J Immunol; 162: 494-502; Aarvak et al. 1999. J Immunol; 162: 1246-1251; Annunziato et al. 1999. J Leukoc Biol; 65: 691-699; Lowes et al. 2008. J Invest Dermatol; 128: 1207-1211). Consequently, several authors have described the characteristic profile of the cytokines of the Th1/Th17 sub-populations, produced by the T cells present in a psoriatic plaque, which provides large quantities of IL-2, IFN-γ, IL-22 and IL-17, and little or no IL-4 and IL-10 (Schlaak et al. 1994. J Invest Dermatol; 102: 145-149; Austin et al. 1999. J Invest Dermatol; 113: 752-759; Wolk et al. 2004. Eur J Immunol 36: 1309-1323; Blauvelt, 2008. J Invest Dermatol; 128: 1064-1067; Lowes et al. 2007. Nature; 445: 866-873 and Nickoloff et al. 2007. Clin Dermatol; 25: 568-573).

On the other hand, several studies (Sano et al. 2005. Nat Med; 11: 43-49; Zenz et al. 2005. Nature; 137: 369-375; Danilenko. 2008. Vet Pathol; 45: 563-575) showed conclusive evidence to indicate that the altered signalling pathways in keratinocytes could play an essential role in the pathogenesis of the disease. Furthermore, recently published genome-wide association studies revealed that a compromised barrier function acts as a key factor in the susceptibility to psoriasis (Zhang et al. 2009. Nat Genet; 41: 205-210; de Cid et al. 2009. Nat Genet; 41: 211-215).

All this notwithstanding, there is a great lack of knowledge about this disease. This lack of knowledge is primarily due to the difficulty in finding an adequate animal model that faithfully reproduces the characteristics of the disease, since the latter only appears in humans, and the generation thereof in experimental animals is deficient. This incapacity to reliably recreate the human disease in animal models lies primarily in the architectural and functional differences between the skin of humans and other animals.

The first approach to be performed was the use of animals that presented spontaneous mutations and showed a phenotype with some characteristics similar to the psoriasis phenotype in human beings. One example is the use of mice with squamous skin (Ttc7^{fsn}/Ttc7^{fsn}) (Beamer et al. 1995. Blood; 86: 3220-3226), which showed hyperproliferation and inflammatory infiltrate, as well as increased vascularisation in the area. These mice had the disadvantage that the aforementioned characteristics were independent from T cells, and, moreover, anti-psoriasis treatments useful in humans did not show efficacy against said elements, which demonstrated that the involved mechanisms had to be different. On the other hand, this phenotype was very complex and presented characteristics different from the human psoriatic phenotype, for which reason this animal model did not make it possible to entirely reproduce the disease.

Other models used to study the disease are genetically modified animals. There are models with alterations, for example, in TGF-β (Li et al. 2004. EMBO J; 23: 1770-1781), STAT-3 (Sano et al. 2005. Nat Med; 11: 43-49), or VEGF (Xia et al. 2003. Blood; 102: 161-168). These animal models have made it possible to study the role of these factors in the psoriatic disorder, but, in general, in an independent manner, not within an adequate context. This is a limitation, since psoriasis is a multi-factor disease and, therefore, the independent study of each factor does not lead to results that are applicable to the disease in humans, although it does contribute to unravel the complex molecular mechanisms underlying the pathology.

These genetic models also include models with alterations in cytokines such as IL-12 or IL-23 (Kopp et al. 2001. J Invest Dermatol; 117: 618-626; Kopp et al. 2003. J Immunol; 170: 5438-5444), which have been useful to understand, at least partially, the involvement thereof in the development of the disease. This has thrown some light into the origin and the mechanisms of action of the disease. Nevertheless, this approach also does not offer all the necessary data to understand the complex development of the pathology.

On the other hand, taking into consideration the presumed involvement of the immune system in the development of the disease, other models have also been developed to attempt to clarify this involvement. To this end, various approaches have also been performed, such as, for example, bone marrow transplants from a psoriatic individual to an immunodepressed individual. This caused lesions in the skin of the recipient individual, which presented a phenotype similar to the psoriatic phenotype. This determined that the T lymphocytes derived from the donour were responsible for the induction of psoriasiform phenotypes and, therefore, that T lymphocytes were involved in the triggering and the development of psoriasis (Snowden and Heaton. 1997. Br J Dermatol; 137: 130-132).

Another study model currently used is the xenotransplantation of skin. This involves transferring skin affected by psoriasis to immunodeficient animals, jointly with the injection of autologous lymphocytes, which makes it possible to incorporate all the elements (both genetic and phenotypic) of psoriasis into the animal model. This approach presents certain problems, such as the need to obtain large quantities of psoriatic skin for the transplants.

Currently, some other models have been developed that bring together, in the same individual, several of the elements present in psoriasis, and which, therefore, provide better conditions for the study of this disease. One of these, for example, is the immunodeficient mouse model, which is administered T lymphocytes and interleukin-12 (W00034459A1). Thus, a psoriatic phenotype is generated with characteristics that are closer to the disease in humans than in the previously described models, although it does not reflect all the elements present in the disease.

Therefore, it has been attempted to generate an animal model that is useful to study the disease and which presents all the characteristics of the psoriatic phenotype in humans by means of different mechanisms. Unfortunately, despite the numerous approaches performed, this objective has not been fulfilled thus far. Therefore, it is necessary to obtain a model that faithfully reproduces, in animal models, the characteristics of psoriasis in humans, in order to be able to perform an in-depth study of the various factors involved in the pathogenesis and the relationships between them, as well as analyse the utility and capacity of various compounds to prevent or treat the disease.

### DESCRIPTION OF THE INVENTION

The present invention relates to a method for inducing a psoriatic phenotype in a non-human mammal, to the animal model of psoriasis obtainable by said method and the use thereof for the identification and evaluation of the efficacy of new treatments.

Psoriasis is a complex multi-factor disease, which, in order to be studied, requires having an animal model that reproduces its characteristics and makes it possible to analyse the various elements that participate in the generation and development thereof.

The present invention offers a solution to the generation of an adequate model for the multi-factor study of psoriasis by reproducing, in an animal, an immunological and epidermal environment similar to that present in the disease in humans.

The present invention discloses a method for the generation of a psoriatic phenotype in animals that may be useful to study the disease, since it reproduces, in the animal, the typical characteristics of the disease in humans, such as epidermal hyperproliferation, elongation and fusion of the epidermal interpapillary ridges, focal acanthosis, parakeratosis, partial loss of the stratum granulosum, inflammatory dermal infiltrate and increased vascularisation of the region. The authors of the present invention demonstrate that performing the steps independently, or combining some of them, does not faithfully reproduce all the characteristics of psoriasis. Therefore, all the steps are essential to generate a useful model for the study of psoriasis.

The process is initiated by the grafting of human cutaneous equivalents or dermal-epidermal substitutes generated by means of tissue engineering. The use of human skin equivalents allows for a better reproduction of psoriasis, since the disease does not naturally appear in non-human animals. Moreover, it allows for a better analysis of the resulting phenotype. Subsequently, activated T lymphocytes of the Th1 sub-population, which have been shown to be involved in the onset, the development and the maintenance of the disease, are intradermally applied in this graft. Cytokines produced by the other sub-population of T lymphocytes involved in the development of the disease (Th17 sub-population) are also applied intradermally in the grafted area. The present invention demonstrates that, in order to generate a complete psoriatic phenotype, it is necessary to use a combination of at least two of these cytokines. Finally, the tape-stripping technique (successive adhesions and removals of a tape) is performed on the skin graft, to produce a disruption of the barrier function of the skin. This entire process generates an environment in the graft that is similar to that produced in regions with psoriatic plaques in humans, thereby allowing for the complete study of the disease.

Therefore, a first aspect of the present invention relates to a method (hereinafter, method of the invention) for inducing a psoriatic phenotype in a non-human mammal, which comprises:
a) Grafting a human skin equivalent in the non-human mammal,
b) Intradermally administering the following, simultaneously or sequentially, in the grafted equivalent of step a):
   - Human T lymphocytes of the Th1 sub-population, and
   - at least two cytokines produced by Th17 lymphocytes, and
c) Applying and removing a tape on the graft of step (a) at least once, to produce a disruption of the epithelial barrier function.

In the present invention, psoriatic phenotype is understood to mean the phenotype that presents characteristics similar to the psoriasis disease in humans, which is **characterised in that** it presents epidermal hyperproliferation, elongation and fusion of the epidermal interpapillary ridges, focal acanthosis, parakeratosis, partial loss of the stratum granulosum, inflammatory dermal infiltrate and increased vascularisation in the region.

In the present invention, phenotype is understood to mean those characteristics that are observable in an organism, and which are determined by its genetic constitution and the environment wherein it lives and develops.

In the present invention, skin equivalent is understood to mean a human bilayer dermal-epidermal substitute, generated *in vitro* and which may be grafted in animals such that it permanently regenerates a skin that is architecturally and functionally analogous to the human skin.

In the present invention, tissue engineering is understood to mean the use of a combination of cells, biochemical factors and/or materials as a function of their biochemical and physical-chemical characteristics to generate tissues that are susceptible to replacing, in whole or in part, any body tissue both structurally and functionally.

In this specification, "animal" is understood to mean any organism of the superkingdom Eukaryota and the kingdom Metazoa. The term "mammal" is used to refer to any organism of the superkingdom Eukaryota, kingdom Metazoa, phylum Chordata, subphylum Craniata, superclass Gnathostomata and class Mammalia. The term human mammal refers to organisms of the superkingdom Eukaryota, kingdom Metazoa, phylum Chordata, subphylum Craniata, superclass Gnathostomata, class Mammalia, order Primates, Family Hominidae, genus Homo and species Homo sapiens.

The function of the embodiment of the tape-stripping technique is to rupture the barrier function of the skin. This is done in order to reproduce the damage which, most often, is the triggering element in the formation of psoriatic plaques. This damage must be sufficient to trigger the response, without generating a damage that compromises the integrity of the skin graft performed. For this reason, in a preferred embodiment of this aspect of the invention, the adhesion and removal of the tape is performed at least 5 times. In a more preferred embodiment of this aspect of the invention, the adhesion and removal of the tape is performed at least 10 times. In an even more preferred embodiment of this aspect of the invention, the adhesion and removal of the tape is performed at least 15 times.

For a correct development of the model, it is necessary that the human skin equivalent undergoes an adequate evolution inside the host organism. To this end, vascularisation and innervation of the grafted region must take place. Moreover, it is necessary that graft-host rejection does not take place. For this reason, it is advisable to use immunodeficient animals to generate the model. Moreover, these immunodeficient individuals allow for a better action of the T lymphocytes administered and a better development of the psoriatic phenotype. For all these reasons, in another preferred embodiment of this aspect of the invention, the non-human mammal is immunodeficient.

In the present invention, immunodeficient individual is understood to mean an organism that presents deficiencies in the immune response, characterised by a numerical and/or functional reduction in T and/or B lymphocytes, and which, therefore, is not capable of rejecting xenotransplants (transplants from one species to another).

In order to generate the human skin equivalents, it is necessary that these equivalents contain cells of human origin which give them this human equivalent characteristic. In general, these equivalents are composed of a dermal matrix formed by one or more elements from the list that comprises, without being limited thereto, collagen, hyaluronic acid and/or fibrin, and which has, jointly to, over or within it, one or more cell types from the list that comprises, without being limited thereto, keratinocytes, melanocytes, Langerhans cells and/or fibroblasts. The most abundant cells, and the most relevant for the generation of these skin equivalents, are keratinocytes, in the epidermis, and fibroblasts, in the dermal region. As a result of the above, in a preferred embodiment of this aspect of the invention, the equivalent is formed by a fibrin matrix, which contains human fibroblasts and keratinocytes. In the present invention, it is demonstrated that fibroblasts and keratinocytes from both healthy individuals, without psoriasis, and individuals with psoriasis are capable of producing the psoriatic phenotype by performing the method of the invention. Therefore, in a preferred embodiment of this aspect of the invention, the fibroblasts and keratinocytes of the human skin equivalent are obtained from a psoriatic patient. In another preferred embodiment, the fibroblasts and keratinocytes of the human skin equivalent are obtained from a healthy individual, without psoriasis.

In the evolution of psoriasis in humans, it has been observed that the lymphocytes that are really involved are T lymphocytes; more specifically, those pertaining to the Th1 and Th17 sub-populations. These sub-populations are defined by the profile of the cytokines that they produce and which lead to various immune responses. The Th1 sub-population is characterised by the secretion of interferon-γ and interleukin-2, and the presence of the CCR5 cytokine receptor. On the other hand, the sub-population of Th17 lymphocytes is characterised by the expression of interleukin-17 and interleukin-22. In order to generate an adequate environment for the evolution of the psoriatic phenotype, it is necessary to intradermally apply T lymphocytes of the Th1 sub-population, and at least two cytokines generated by the other sub-population of lymphocytes involved, Th17 lymphocytes, in the graft performed. The cytokines produced by the Th17 lymphocyte sub-population that have been proven to be the most important in the evolution of psoriasis are interleukins IL-17 and IL-22. According to the data shown in the present specification, the administration of interleukin-22, jointly with the rest of the steps, already generates a hyperproliferation model that is useful for the study thereof. However, the additional administration of IL-17 generates a more complete model of psoriasis. For this reason, in a preferred embodiment of this aspect of the invention, the cytokines that are administered are interleukin-22 and interleukin-17.

As is well-known, the most widely used animal models for the study of diseases are those performed in rodents, primarily mice. This is primarily due to the reduced space that these animals require, as compared to other, larger-size animals, and to the ease of rearing and handling thereof. For this reason, in a preferred embodiment of this aspect of the invention, the non-human mammal is a rodent. In a more preferred embodiment of this aspect of the invention, the mammal is a mouse.

Taking into consideration that immunodeficient animals show a better response to xenotransplants and the administration of lymphocytes, and the greater ease of handling of mice, it is necessary to use immunodeficient mice to generate the animal model. The most widely used immunodeficient mice in experimentation are either nude mice, which present thymic aplasia and, therefore, deficiencies in the development of lymphocytes, or mice with severe combined immunodeficiency (SCID). NOD-SCID mice (non-obese diabetic mice with severe combined immunodeficiency) are mice that do not present T or B lymphocytes, and, moreover, have lymphopenia and hypogammaglobulinaemia, for which reason their immune response is deficient. On the other hand, NMRI Foxn1^{nu} mice are mice that present thymic aplasia due to deficiencies in the development of the thymic epithelium. Due to this deficiency in thymic development, they present deficiencies in the generation of immune cells and, therefore, they also have a reduced immune response. For all these reasons, in an even more preferred embodiment of the present invention, the mouse used is an immunodeficient NMRI Foxn1^{nu} or NOD-SCID mouse.

Another aspect of the present invention relates to the animal model generated by means of the method of the invention.

Since it presents the characteristics of psoriasis in humans, the animal model generated is very useful for the study of the disease, as well as for the search of treatments designed to attenuate the symptoms or cure the disease. This, which is aimed at improving the quality of life of persons, clearly justifies the possible suffering caused to the animal in generating the model. For all these reasons, another aspect of the invention relates to the use of the model generated by means of the method of the invention for the identification of a compound or composition for the prevention or treatment of psoriasis. Another aspect of the invention relates to the use of the model to evaluate the efficacy of a preventive or therapeutic treatment against psoriasis.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following examples and drawings are provided for illustrative purposes, and are not intended to limit the scope of the present invention.

### DESCRIPTION OF THE FIGURES

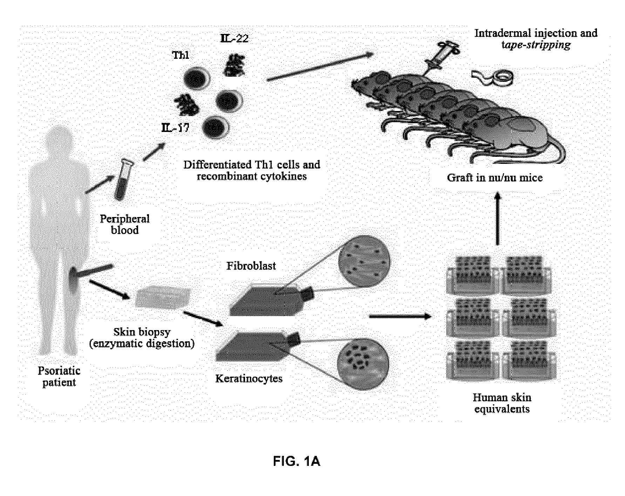

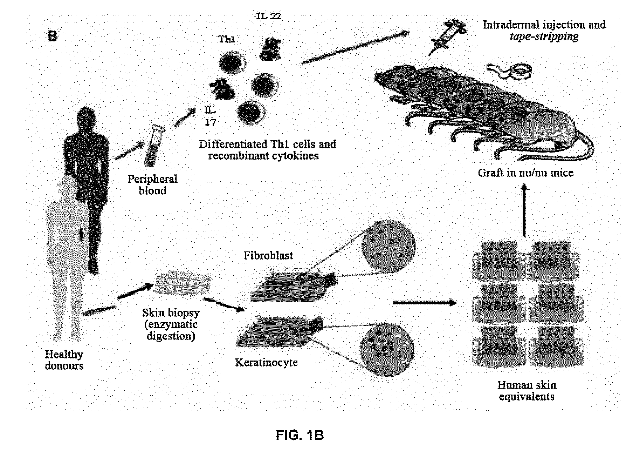

Figure 1 shows a schematic diagram of the experimental design for the generation of the animal model of psoriasis.

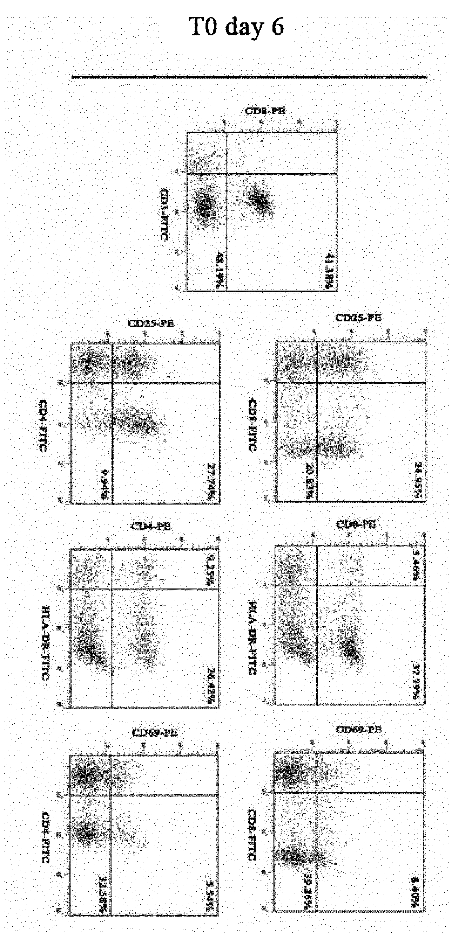

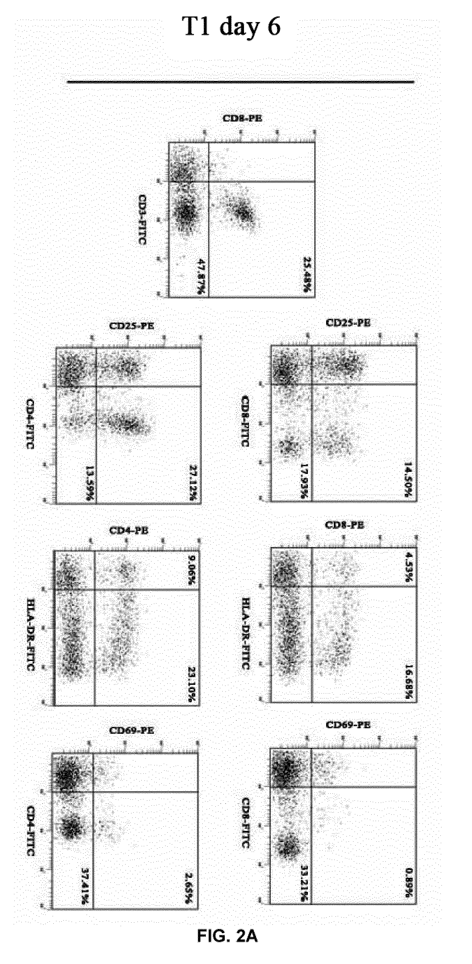

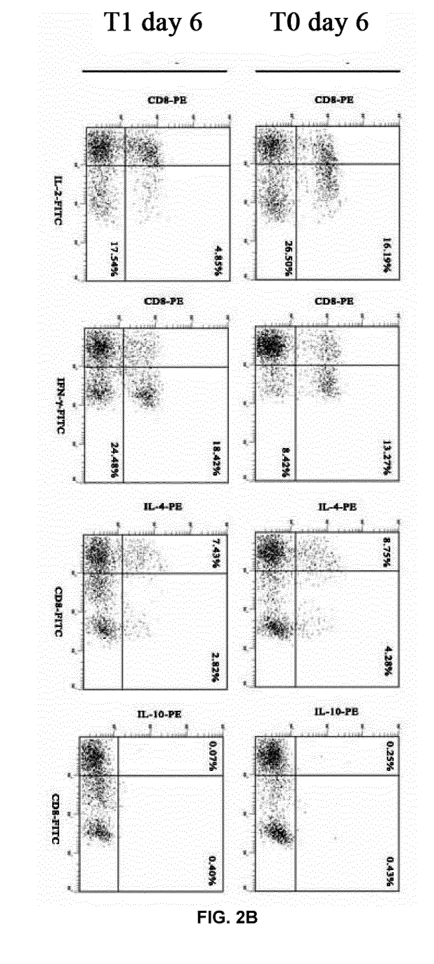

Figure 2 shows the analysis by flow cytometry of the sub-populations of T lymphocytes differentiated *in vitro.* PBLs (peripheral blood lymphocytes) from healthy donours were cultured with Dynabeads CD3/CD28 T Cell Expander (1:1 ratio) and 30 U/ml of human interleukin-2. Cells were cultured for 6 days under these conditions (T0), and IL-12 and anti-IL-4 were added to the culture (T1) in order to induce differentiation. The analyses by flow cytometry were performed on the sixth day of differentiation. A) Representative dot plots of the surface markers in the T0 and T1 sub-populations differentiated *in vitro.* B) Dot plots of the staining patterns of intracellular cytokines. The percentages are relative to the proportion of positive cells defined by the binding of control antibodies.

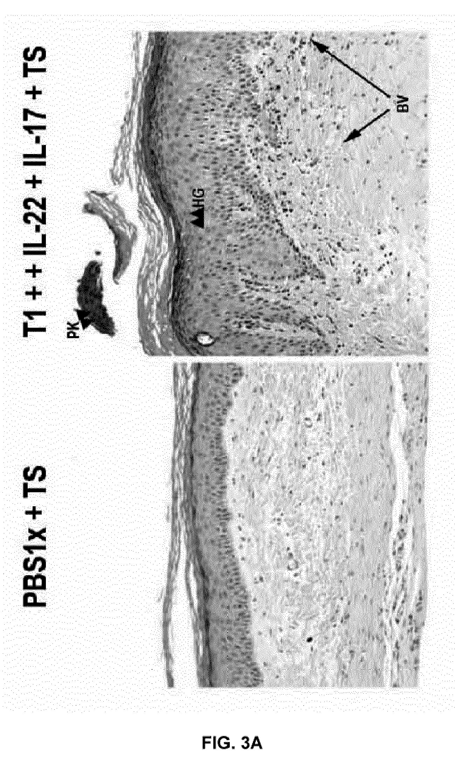

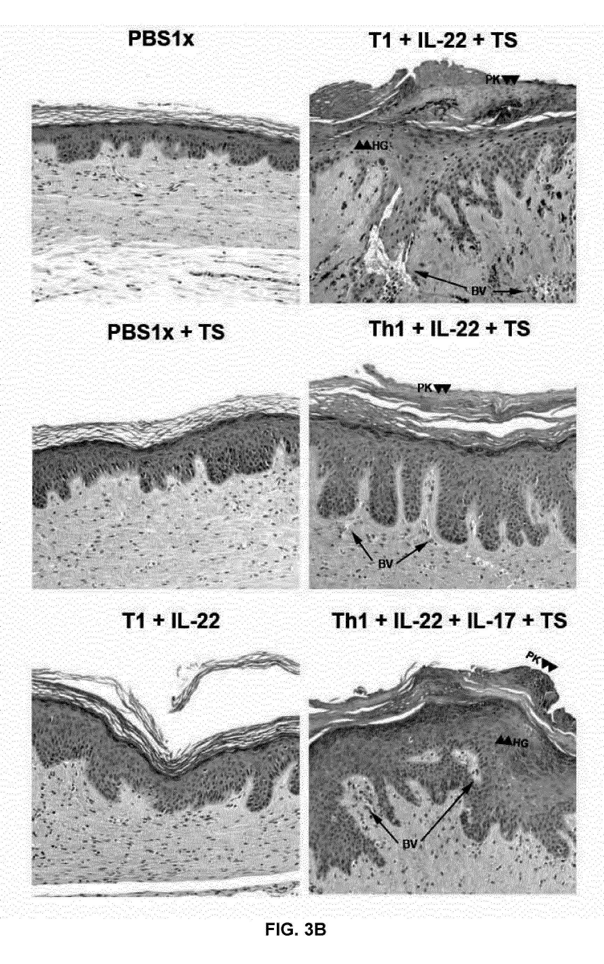

Figure 3 shows the histological analysis of the human skin regenerated following the administration of lymphocytes and cytokines. The staining with haematoxylin/eosin was performed in sections, fixed in formalin and embedded in paraffin, of human grafts that were intradermally injected with differentiated lymphocytes of the Th1 sub-population, and/or with recombinant IL-22 and IL-17. Tape-stripping (TS) was performed where indicated. Areas of hypogranulosis (HG) and parakeratosis (PK) were observed when the graft was injected with recombinant cytokines jointly with T lymphocytes, and tape-stripping was applied. The arrows indicate the presence of dilated capillaries in the dermis (BV).

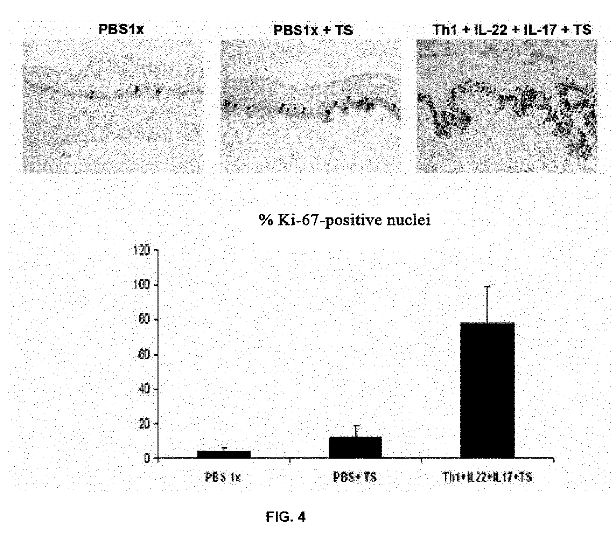

Figure 4 shows the proliferative response to the dermal injection and/or to tape stripping in regenerated human skin. The formalin-fixed sections were stained for proliferation marker Ki-67. The proliferation index was calculated by the percentage of Ki-67-positive nuclei for every 100 nuclei of the basal layer, in several randomly selected areas.

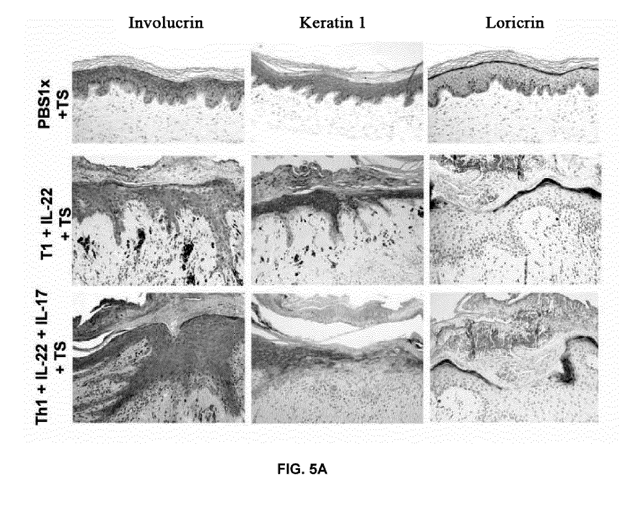

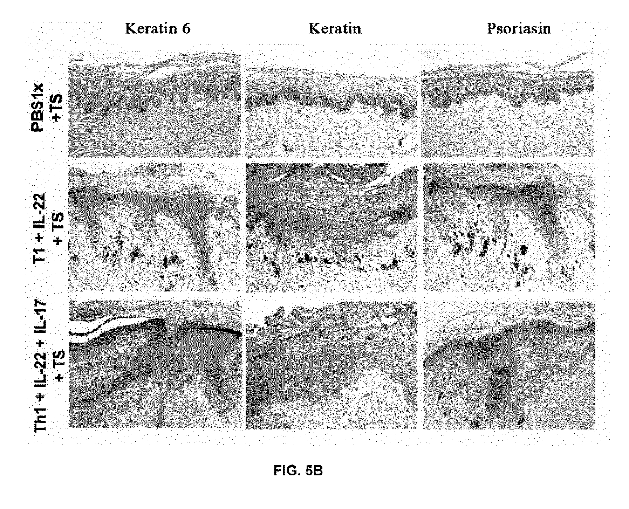

Figure 5 shows the immunohistochemical analysis of epidermal markers. Consecutive sections, fixed with formalin and embedded in paraffin, are used in Figures 3 and 4. A) These are stained for differentiation markers involucrin and loricrin, as well as for keratin-1. B) The staining of hyperproliferation markers keratin-6 and keratin-17, and of psoriasin (hS100A7), was also performed on consecutive sections.

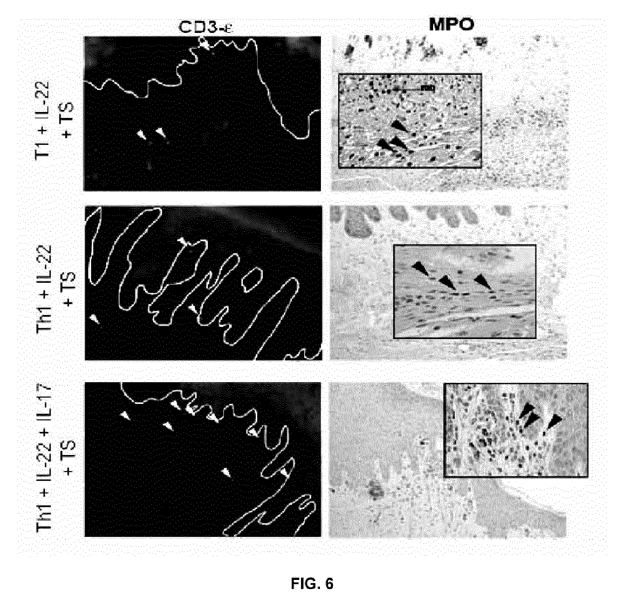

Figure 6 shows the immunohistochemical and immunofluorescence analyses of dermal cells. The composition of the inflammatory infiltrate was analysed by the immunohistochemical analysis of consecutive sections of tissue embedded in paraffin and fixed with formalin, using an anti-myeloperoxidase antibody (MPO) to detect cells from the granulocytic series. Immunofluorescence analysis for the T-cell-specific CD3-ε antigen in frozen sections indicated the presence of the T cells injected in the cryostat sections obtained from tissue samples adjacent to those obtained for the immunohistochemical analyses.

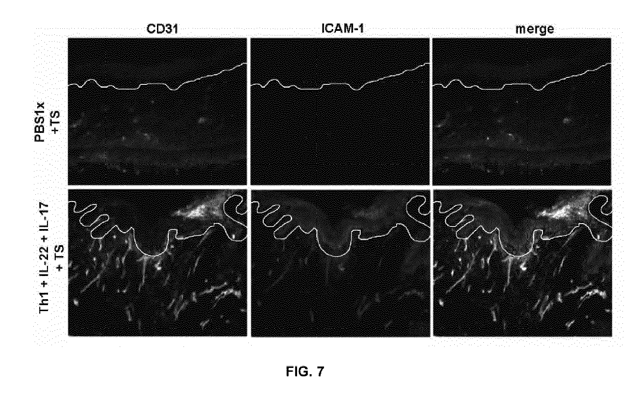

Figure 7 shows the immunofluorescence analysis of the angiogenic tissue reaction. The double immunofluorescence analysis for the endothelial-cell-specific CD31 antigen and intercellular adhesion molecule 1 (ICAM-1) are shown.

### EXAMPLES

The following specific examples provided in this patent document serve to illustrate the nature of the present invention. These examples are included solely for illustrative purposes and should not be interpreted as limitations to the invention claimed herein. Therefore, the examples described further below illustrate the invention without limiting the field of application thereof.

### EXAMPLE 1. Experimental design for the generation of a model of humanised skin with a psoriatic phenotype

The contribution of the components (epidermal and immune) to the pathogenesis of the disease was considered to be a critical factor for the experimental design presented in this document, in order to imitate the human ailment as closely as possible. To this end, the set of T1 lymphocytes was obtained from peripheral blood using *in vitro* cytokine-directed polarisation. These immune cells were re-introduced into the mature skin of a skin-humanised mouse model by means of intradermal injection, jointly with the recombinant cytokines of the Th17 sub-population, IL-17 and IL-22. The humanised-skin mouse model was generated by obtaining keratinocytes and fibroblasts, by enzymatic digestion, from human skin biopsies of both a psoriatic patient (Figure 1 A) and a healthy donour (Figure 1B). The cells were amplified under culture and assembled in a fibrin-based organotypical culture that was grafted on the back of immunodeficient mice using a system previously characterised in our laboratory (Del Rio et al. 2002. Hum Gene Ther; 13: 959-968; Llames et al. 2004. Transplantation; 77: 350-355). This system makes it possible to obtain a large number of mice grafted with a significant area of skin from a single donour, which is one of the main advantages of this model as compared to other humanised models, such as the xenotransplantation model (Boehcke et al. 1996. Nature; 379: 777; Wrone-Smith and Nickoloff 1996. J Clin Invest; 98: 1878-1887). Moreover, the barrier function of the skin was compromised by using the tape-stripping technique, a well-characterised process designed to eliminate the superficial layers of the corneal stratum, which produces hyperproliferation without severe inflammation (Ahn et al. 1999. J Invest Dermatol; 113: 189-195).

### EXAMPLE 2. In vitro differentiation of the sub-populations of T1 cells

The authors differentiated T cells *in vitro* towards a type 1 phenotype by means of cytokine-directed activation and polarisation. To this end, PBLs (peripheral blood lymphocytes) from psoriatic patients or healthy donours, obtained by density gradient separation, were activated, using a combination of CD3/CD28 antibodies conjugated with magnetic beads, in the presence of IL-2. The differentiation towards Th1 and Th2 is controlled by means of IL-12 (p35-p40) and IL-4, respectively. Moreover, it is well-known that these cytokines inhibit the generation of the opposite Th subset. Therefore, the authors used a well-established process to obtain the cytokine-directed polarisation of Th1, culturing the activated T lymphocytes for 6 days in the presence of IL-12 and anti-IL-4. The T0 cells corresponded to T lymphocytes activated under culture in the presence of IL-2 alone. On day 6, the proportion of CD3+ cells varied between 70 % and 90 %, depending on the donour, with a CD4:CD8 ratio of 1.2-1.8. The activation state was evaluated by means of the expression of CD25 (IL-2Rα), HLA-DR and CD69 on the cellular surface. The FACS profiles corresponding to a healthy donour are shown in Figure 2A. In this case, following 6 days of culture, a high percentage of CD4+ cells also expressed CD25 (66.61 % of CD4+ T1 cells vs. 73.61 % of CD4+ T0 cells). The proportion of CD8+ cells that jointly expressed CD25 was lower (44.71 % of CD8+ T1 cells vs. 54.49 % of CD8+ T0 cells). In both populations of CD4+ and CD8+ T cells, a high proportion of cells expressed HLA-DR under T0 or T1 polarisation conditions (78.64 % of CD8+ T1 cells vs. 91.61 % of CD8+ T0 cells, and 71.82 % of CD4+ T1 cells vs. 74.06 % of CD4+ T0 cells). On the contrary, the early activation marker showed low levels of expression, especially under Th1 polarisation conditions (6.61 % of CD4+ and 2.60 % of CD8+ in T1 cells vs. 14.52 % of CD4+ and 17.62 % of CD8+ in T0 cells).

The cytokine profile of the in vitro differentiated T cells was evaluated by means of flow cytometry in the CD4+ and CD8+ T-cell sub-populations following their stimulation with phorbol myristate acetate (PMA) and ionomycin. On day 6 of T1 differentiation, a high percentage of cells expressed IFN-γ (42.9 % as compared to 21.69 % expressed by T0 cells on day 6) when they were activated. A small proportion of cells expressed IL-2 (22.39 % as compared to 42.69 % T0 cells that presented expression) and only a small proportion of T1 and T0 cells expressed IL-4 and IL-10 (Figure 2B). The T1 cells also produced large quantities of GM-CSF (granulocyte macrophage colony stimulating factor), as evaluated by means of a specific ELISA assay (data not shown).

### EXAMPLE 3. Intradermal injection of differentiated T1 lymphocytes and recombinant cytokines in the model of humanised skin

The histological analyses showed that the injection of T1 lymphocytes, jointly with recombinant IL-22 and tape-stripping, induced the typical epidermal changes associated with psoriasis, including elongation and fusion of the interpapillary ridges, focal acanthosis, parakeratosis and partial loss of the granular layer. The dermis is characterised by a slight inflammatory infiltrate and an increase in vascularisation, with the presence of dilated capillaries. When recombinant IL-17 was added to the aforementioned combination, an even more intense inflammatory dermal response was observed in the presence of this cytokine. This reaction was observed both within an autologous context, where the regenerated skin and the immune cells were from a psoriatic patient (Figures 1A and 3A), and, more importantly, within an allogeneic context, where the regenerated skin and the immune cells were from unrelated healthy donours (Figures 1B and 3B). In this case, in order to exclude an adverse reaction due to allogeneic recognition, the population of T1 lymphocytes from CD8+ cells was depleted, using magnetic beads, prior to each injection. In this case, as in the preceding case, the main characteristics of the psoriatic phenotype were present. On the contrary, the injection of T1 lymphocytes, jointly with recombinant IL-22 alone, did not induce the psoriatic phenotype in the absence of tape-stripping, and only a slight epidermal hyperplasia reaction was observed. Similarly, tape-stripping by itself did not induce a psoriasiform reaction (Figure 3B). Immunostaining with Ki-67 revealed an increase in the number of Ki-67-positive cells in the epidermis of skin grafts injected with IL-22 alone, T1 cells plus IL-22 (data not shown) or tape-stripping alone (Figure 4), which increased further when T1 cells plus IL-22/IL-17 were jointly administered in the presence of tape-stripping. In this case, the positive cells were not restricted to the basal layer, but included suprabasal cells (Figure 4).

Some of the anomalies found in the psoriatic lesions were identified by immunohistochemical analysis of some keratinocyte differentiation markers (expression of involucrin, loricrin and keratin). The simultaneous injection of T1 cells plus the IL-22 and IL-17 recombinant cytokines in the skin grafts, jointly with tape-stripping, is the condition that most resembles the immunohistochemical features of human psoriasis. Involucrin seems to be overexpressed, whereas the expression of loricrin was lower in those areas where the generation of a well-differentiated granular layer was hindered. The expression of keratin K1 was also disturbed, with a focal inhibition of the expression thereof, whereas a clear overexpression of K6 and K17 coincided with the presence of a hyperproliferative epidermis using this condition. Moreover, the expression of antimicrobial protein S100A7 (psoriasin) was also induced (Figures 5A and B).

A more prominent inflammatory infiltrate was observed when recombinant IL-17 was simultaneously administered with skin grafts injected with T1 plus IL-22 in the presence of tape-stripping (Figures 3A and B). The immunohistochemical analyses to evaluate the cellular composition of the infiltrate revealed an increased influx of granulocytes and macrophages at the injection point (Figure 6). The analysis using anti-CD3 specific antibodies showed the location of the T1 cells injected in the humanised skin grafts (Figure 6). Immunofluorescence with CD31 showed the presence of dilated capillaries in the dermis of the humanised skin grafts simultaneously injected with IL-22 and IL-17 plus Th1 in the presence of tape-stripping, and this is correlated with an increased expression of ICAM-1 in the vessels (Figure 7).

### MATERIALS AND METHODS USED

### Patients

All patients included in this study presented the psoriasis vulgaris variant of the disease, with an early onset and high PASI (Psoriasis Area Severity Index) scores. Some of them also showed some case of psoriasis in their family history. When the skin biopsies were taken, the medication had been interrupted for several months. Samples were taken, with local anaesthesia, of both asymptomatic skin and psoriatic plaques using a 6-mm biopsy needle. The psoriatic patients were recruited at the Hospital Básico de la Defensa (Valencia, Spain) and signed an informed consent. YCP: Years with diagnosed psoriasis; PFH: Psoriasis family history; PASI: Psoriasis Area Severity Index; BSA: Body surface affected.

**Table 1: Psoriatic patients participating in the study**

| Patient number | Age (years) | Weight (kg) | YCP | PFH | PASI | BSA |
|---|---|---|---|---|---|---|
| 1 | 51 | 72 | | | | |
| | | | 6 | No | 10 | 5.84 |
| 2 | 46 | 83 | | | | |
| | | | 20 | Yes | 12 | 8.35 |
| 3 | 56 | 84 | | | | |
| | | | 28 | Yes | 12 | 22.86 |
| 4 | 29 | 70 | | | | |
| | | | 20 | No | 10.4 | 11.43 |
| 5 | 60 | 85 | | | | |
| | | | 20 | No | 18 | 17.68 |
| 6 | 78 | 90 | | | | |
| | | | 3 | No | 20.4 | 23.2 |

### Isolation of lymphocytes and expansion of T cells

Peripheral blood from both psoriatic patients and HIV-seronegative donours (provided by the Centre for Transfusions, Madrid, Spain) was used to isolate peripheral blood mononuclear cells (PBMC) by means of Ficoll-Hypaque density gradients (Pharmacia, Piscataway, NJ). Cells were cultured in RPMI 1640, supplemented with 10 % heat-inactivated foetal bovine serum (FCS), and stimulated with Dynabeads CD3/CD28 T Cell Expander® (1:1 ratio) and 30 U/ml of human interleukin-2 (R&D Systems Inc., Minneapolis, MN) for 4-6 days. For the differentiation to T1, IL-12 (20 ng/ml) (R&D Systems Inc.), jointly with anti-IL-4 antibody (5 pg/ml) (BD Pharmingen, San Diego, CA), were also added to the culture. The Th1 cells were obtained by negative selection of these cultures using the CD4+ T lymphocyte isolation kit from Miltenyi Biotec (Auburn, CA).

### Analysis by flow cytometry

A phenotype analysis of the lymphocyte sub-populations was performed, before and after expansion T cells, by means of flow cytometry (FCM), using an EPICS cytofluorimeter (Beckman Coulter, Fullerton, CA). Cells were cultured, washed and suspended in phosphate-buffered saline solution with 1 % bovine serum albumin (Sigma-Aldrich). Aliquots (2 x 10⁵ cells) were incubated in the dark at 4 °C (30 minutes) with conjugated monoclonal antibodies and washed (human anti-CD3, CD4, CD8, CD25, CD69, HLA-DR monoclonal antibodies (Becton, Dickinson and Company, San Jose, CA)). The non-specific fluorescence was determined using anti-isotype monoclonal antibodies. For the intracellular staining, monensin (GolgiStop from Pharmingen) was added at the end of the 4 h of activation of the T cells. Subsequently, the cells were fixed in 4 % paraformaldehyde for 10 minutes and permeabilised with 0.1 % saponin. The staining was performed with the anti-IFNγ, anti-IL-2, anti-IL-4 and anti-IL-10 antibodies (conjugated with PE (phycoerythrin) or FITC (fluorescein isothiocyanate), Pharmingen) in 0.1 % saponin. The cells were washed and subjected to analysis by fluorescence flow cytometry (FACS, or Fluorescence Activated Cell Sorter).

### Primary cultures of human keratinocytes and fibroblasts

The human dermal keratinocytes and fibroblasts were obtained by enzymatic digestion from skin biopsies performed on donours (Rheinwald and Green 1975. Cell; 3: 331-343). The cultures were performed following ethical approval and having previously obtained the donours' informed consent. The study was performed in accordance with the Declaration of Principles of Helsinki. The primary keratinocytes were cultured on a nourishing layer of lethally irradiated 3T3-J2 cells (X-rays; 50 Gy) (donation from Dr. J. Garlick, SUNY, Stony Brook, NY), as previously described (Meana et al. 1998. Burns; 24: 621-630; Del Rio et al., 2002. Hum Gene Ther, 13: 959-968). The keratinocyte seeding medium was a 3:1 mixture of Dulbecco's Modified Eagle Medium (DMEM) (GIBCO-BRL, Gaithersburg, MD) and Ham's F12 (GIBCO-BRL) containing 10 % foetal bovine serum (FCS), 0.1 nM cholera toxin, 2 nM triiodotyrosine (T3), 5 µg/ml insulin, 0.4 µg/ml of hydrocortisone and 10 µg/ml of EGF (Sigma, St Louis, MO). The primary fibroblasts were cultured on plastic in DMEM containing 10 % FCS. The cells were cultured at 37 ºC in a humid atmosphere containing 5 % CO₂. The culture medium was changed every 2 days.

### Animals

Immunodeficient Rj: NMRI-Foxn1 ^{nu} (NMRI nu) mice (6-8 weeks of age) were used (Elevage Janvier, Le Genest Saint Isle, France). During the experiment, the mice were housed at the CIEMAT's Laboratory Animal Facility (Spanish registration number 28079-21 A) under pathogen-free conditions, using IIL-type cages individually ventilated with microinsulators, with a maximum of six mice per cage, with 25 air changes per hour and heat-treated soft wood briquettes for the beds. All the experimental processes were performed in accordance with the Spanish and European legislation, and following the regulations on the protection and use of animals in scientific research. The processes were approved by the authors' Animal Experimentation Ethics Committee in accordance with all the internal and external biosafety and bioethics guidelines.

### Human skin equivalents designed by tissue engineering and grafted

The biodesigned human skin equivalent is based on the use of live fibroblasts contained in a fibrin matrix as the dermal component (Meana et al. 1998. Burns; 24: 621-630). In order to generate it, 1.5 ml of fibrinogen solution (from cryoprecipitated pig blood) were added to 5 ml of keratinocyte growth medium containing 2.5 x 10⁵ dermal fibroblasts and 250 IU of bovine aprotinin (Trasylol; Bayer, West Haven, Connecticut). Immediately thereafter, 0.5 ml of 0.025 mM Cl₂Ca were added, with 5.5 IU of bovine thrombin (Sigma-Aldrich Co, St. Louis, MO). The mixture was placed in a 6-well culture plate (Corning Costar Corp., Cambridge, MA) and allowed to solidify at 37 ºC for 2 hours. The keratinocytes were seeded on this matrix and grown in submerged culture until confluence was reached. The mice were aseptically cleaned and transplanted following the method previously described in our laboratory (Del Rio et al. 2002. Hum Gene Ther; 13: 959-968; Llames et al. 2004. Transplantation; 77: 350-355; Serrano et al. 2003. Hum Gene Ther; 14: 1579-1585).

### Intradermal injection of the sub-populations of T lymphocytes and recombinant cytokines

Between nine and twelve weeks after the transplantation, the *in-vitro-*derived sub-populations of T lymphocytes (10⁶/50µl), or the recombinant cytokines (200 ng/50µl) diluted in sterile PBS, were inoculated, by intradermal injection, in the skin with the stable human graft every two days for 8 days. In some cases, prior to the injection, the tape-stripping technique was applied 15 times on the same transplantation area. The mice were sacrificed by carbon dioxide asphyxiation two days after the last intradermal injection and skin biopsies were taken, which were processed in order to perform the histological and immunohistochemical analyses.

### Histological and immunohistochemical analyses

Paraffin sections fixed with formalin (4-6 µm) were deparaffinised by melting for 30-60 min at 60 ºC, cleaned in xylene three times for 5 min and rehydrated in aqueous solutions containing decreasing percentages of ethanol. In order to determine the tissue architecture, the sections were stained with haematoxylin-eosin (Gill's Haematoxylin 2 and alcoholic Eosin Y; Thermo Sandon, Cheshire, UK) following the standard process.

For the staining with immunoperoxidase, the sections were treated in order to de-activate the endogenous peroxidase, blocked and incubated overnight at 4 ºC with specific primary antibodies against human epidermal and granulocyte markers. The antibodies were used at final dilutions of 1:500 and 1:300 for the anti-keratin-1 and 17 antibodies, respectively (Sigma Aldrich), 1:1000 for the anti-keratin-6 monoclonal antibody (clone LHK6B, Neomarkers, Fremont, CA), 1:2000 for the anti-loricrin polyclonal antibody (Babco, Richmond, CA) and 1:50 for the anti-myeloperoxidase antibody (MPO) (HyCult biotechnology b.v., Uden, Netherlands). In order to establish the human origin of the regenerated skin, a specific antibody against human involucrin (clone SY5; Sigma-Aldrich, St Louis, MO) was used to label the human keratinocytes. The cell proliferation was evaluated by means of immunoperoxidase detection of the Ki-67 antigen, using a rabbit monoclonal antibody (Clone SP6, Neomarkers). The biotynilated specific secondary antibodies for each case were obtained from Jackson ImmunoResearch Laboratories (West Grove, PA). All the stainings with immunoperoxidase were performed with standard processes using the Vectastain ABC kit (Vector Laboratories Inc., Burlingame, CA). The sections were counterstained with haematoxylin and dehydrated in an aqueous solution containing increasing percentages of ethanol. Finally, the plates were incubated for 15 minutes in Histoclear (National Diagnostic, Atlanta, GA) and mounted. Images were taken with an Olympus Bx41 microscope with a digital camera.

The immunofluorescence analyses were performed on 8-10 µm cryostat sections obtained from tissue regions adjacent to those mentioned in the preceding paragraph, which were embedded in OCT (optimal cutting temperature) medium (TissueTek). In order to analyse the vascular density, sections fixed in cold acetone were used. The plates were incubated with an anti-CD31 monoclonal antibody (PECAM-1) (clone MEC 13.3, Pharmingen) diluted to 1:100. Double immunofluorescence was performed with an anti-ICAM-1 polyclonal antibody diluted to 1:50 (Santa Cruz Biotech, Santa Clara, CA). Consecutive sections were stained with the anti-CD3ε polyclonal antibody (Dako, Glostrup, Denmark). The FITC- or Texas-Red-coupled secondary antibodies were purchased from Jackson ImmunoResearch Laboratories.

## Claims

1. Method for inducing a psoriatic phenotype in a non-human mammal, which comprises:
a) Grafting a human skin equivalent in the non-human mammal,
b) Intradermally administering the following, simultaneously or sequentially, in the grafted equivalent of step a):
• Human T lymphocytes of the Th1 sub-population, and
• at least two cytokines produced by Th17 lymphocytes, and
c) Applying and removing, at least once, a tape on the graft of step (a), to produce a disruption of the epithelial barrier function.

2. Method according to claim 1, where in step (b) the human T lymphocytes of the Th1 sub-population and at least two cytokines produced by Th17 lymphocytes are simultaneously administered.

3. Method according to claims 1 or 2, where the non-human mammal is immunodeficient.

4. Method according to any of claims 1 to 3, where the skin equivalent used to perform the graft of step (a) is a fibrin matrix with human fibroblasts and keratinocytes.

5. Method according to claim 4, where the fibroblasts and keratinocytes of the human skin equivalent are from a psoriatic patient.

6. Method according to claim 4, where the fibroblasts and keratinocytes of the human skin equivalent are from a healthy individual.

7. Method according to any of claims 1 to 6, where the cytokines of step (b) are interleukin-17 and interleukin-22.

8. Method according to any of claims 1 to 7, where the non-human mammal is a rodent.

9. Method according to claim 8, where the rodent is a mouse.

10. Method according to claim 9, where the mouse is an immunodeficient NMRI-Foxn1 ^{nu} or NOD-SCID mouse.

11. Non-human animal model that shows a psoriatic phenotype obtainable using the method according to any of claims 1 to 10.

12. Use of the model according to claim 11, for the identification of a compound or composition for the prevention or treatment of psoriasis.

13. Use of the model according to claim 11, to evaluate the efficacy of a preventive or therapeutic treatment against psoriasis.
